# EUROPÄISCHE PATENTSCHRIFT

(11) **EP 1 318 406 B1**
(45) Veröffentlichungstag und Bekanntmachung des Hinweises auf die Patenterteilung: **09.11.2005**
(21) Anmeldenummer: 01128852.9
(22) Anmeldetag: 04.12.2001
(51) Int. Cl.: G01N 33/68, G01N 33/569, A61P 31/04, A61P 37/02

(54) **Verwendung der Glycin-N-Acyl-Transferase (GNAT) für die Diagnose von Entzündungserkrankungen und Sepsis**
Use of Glycine-N-Acyl-Transferase (GNAT) for diagnosis of inflammatory diseases and sepsis
Utilisation de la Glycine-N-Acyl-Transférase (GNAT) pour le diagnostic des maladies inflammatoires et de la septicémie

(43) Veröffentlichungstag der Anmeldung: 11.06.2003
(73) Patentinhaber: B.R.A.H.M.S Aktiengesellschaft, 16761 Hennigsdorf (DE)
(72) Erfinder: Bergmann, Andreas, Dr., 12351 Berlin (DE); Struck, Joachim, Dr., 12161 Berlin (DE); Ühlein, Monika, Dr., 10407 Berlin (DE)
(74) Vertreter: Andrae, Steffen

(56) Entgegenhaltungen:
- EP-A- 1 077 258
- WO-A-01/75067
- REINHART, K ET AL: "Intensivmedizin: Sepsis und septischer Schock (Seiten 756-760)" 2001 , THIEME VERLAG, XP008003130 * Seite 756, rechte Spalte, Absatz 3 *
- BEISHUIZEN A ET AL: "Endogenous mediators in sepsis and septic shock" ADVANCES IN CLINICAL CHEMISTRY, Bd. 33, 1999, Seiten 55-131, XP008003176

### SEQUENCE LISTING

<110> B.R.A.H.M.S Aktiengesellschaft
<120> Verwendungen der Gylcin-N-Acyl-Transferase (GNAT) für die Diagnose und Therapie von Entzündungserkrankungen und Sepsis
<130> 3679 AS
<140>
   <141>
<160> 2
<170> PatentIn Ver. 2.1
<210> 1
   <211> 8
   <212> PRT
   <213> Primat (Pavian)
<400> 1
   Met Leu Pro Leu Gln Gly Ala Gln
    1               5
<210> 2
   <211> 296
   <212> PRT
   <213> Homo sapiens
<300>
<400> 2
   Met Met Leu Pro Leu Gln Gly Ala Gln Met Leu Gln Met Leu Glu Lys
    1               5                  10                  15
   Ser Leu Arg Lys Ser Leu Pro Ala Ser Leu Lys Val Tyr Gly Thr Val
               20                  25                  30
   Phe His Ile Asn His Gly Asn Pro Phe Asn Leu Lys Ala Val Val Asp
           35                  40                  45
   Lys Trp Pro Asp Phe Asn Thr Val Val Val Cys Pro Gln Glu Gln Asp
       50                  55                  60
   Met Thr Asp Asp Leu Asp His Tyr Thr Asn Thr Tyr Gln Ile Tyr Ser
   65                  70                  75                  80
   Lys Asp Pro Gln Asn Cys Gln Glu Phe Leu Gly Ser Pro Glu Leu Ile
                   85                  90                  95
   Asn Trp Lys Gln His Leu Gln Ile Gln Ser Ser Gln Pro Ser Leu Asn
               100                 105                 110
   Glu Ala Ile Gln Asn Leu Ala Ala Ile Lys Ser Phe Lys Val Lys Gln
           115                 120                 125
   Thr Gln Arg Ile Leu Tyr Met Ala Ala Glu Thr Ala Lys Glu Leu Thr
       130                 135                 140
   Pro Phe Leu Leu Lys Ser Lys Ile Leu Ser Pro Ser Gly Gly Lys Pro
   145                 150                 155                 160
   Lys Ala Ile Asn Gln Glu Met Phe Lys Leu Ser Ser Met Asp Val Thr
                   165                 170                 175
   His Ala His Leu Val Asn Lys Phe Trp His Phe Gly Gly Asn Glu Arg
               180                 185                 190
   Ser Gln Arg Phe Ile Glu Arg Cys Ile Gln Thr Phe Pro Thr Cys Cys
           195                 200                 205
   Leu Leu Gly Pro Glu Gly Thr Pro Val Cys Trp Asp Leu Met Asp Gln
       210                 215                 220
   Thr Gly Glu Met Arg Met Ala Gly Thr Leu Pro Glu Tyr Arg Leu His
   225                 230                 235                 240
   Gly Leu Val Thr Tyr Val Ile Tyr Ser His Ala Gln Lys Leu Gly Lys
                   245                 250                 255
   Leu Gly Phe Pro Val Tyr Ser His Val Asp Tyr Ser Asn Glu Ala Met
               260                 265                 270
   Gln Lys Met Ser Tyr Thr Leu Gln His Val Pro Ile Pro Arg Ser Trp
           275                 280                 285
   Asn Gln Trp Asn Cys Val Pro Leu
       290                 295

## Beschreibung

Die vorliegende Erfindung betrifft neue Verwendungen des Enzyms Glycin-N-Acyl-Transferase (nachfolgend stets abgekürzt GNAT) für die medizinische Diagnostik von Entzündungserkrankungen und Sepsis. Sie beruht auf dem erstmaligen Nachweis stark erhöhter Konzentrationen der GNAT in Lebergewebe von Primaten, bei denen experimentell durch Toxinverabreichunge eine Sepsis bzw. systemische Entzündung erzeugt worden war.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen, insbesondere von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Rein-hart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund, ohne dass dessen Rolle in der komplexen Kaskade der am Entzündungsgeschehen beteiligten endogenen Substanzen bekannt sein muss.

Eine derartige als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zu Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können, signifikant erhöhte Konzentrationen beobachtet werden können. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird ein Ergebnis eines anderen fruchtbaren, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procalcitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene sepsisspezifische Biomarker von peptischer bzw. proteinischer Natur zu ermitteln, deren Auftreten für Sepsis oder bestimmte Formen von Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Diese therapeutischen Ansätze laufen darauf hinaus, die Konzentrationen entzündungsfördernder Substanzen zu senken bzw. die Entstehung derartiger Substanzen zu hemmen, und zwar insbesondere unter Verwendung von spezifischen Antikörpern (gegen TNF-α bzw. MIF; vgl. E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170) bzw. die Konzentration von hemmend in die Entzündungskaskade eingreifenden endogenen Substanzen (Protein C; K.Garber, "Protein C may be sepsis solution", Nature Biotechnology, Vol.18, 2000, 917-918) zu erhöhen. In der letztgenannten Veröffentlichung findet sich ein Überblick über derartige, leider bisher meist wenig erfolgreiche Versuche, das Entzündungsgeschehen unter Beeinflussung ausgewählter endogener Target-Moleküle therapeutisch zu beeinflussen. Angesichts der bisherigen eher enttäuschenden therapeutischen Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Die vorliegenden Erfindung beruht darauf, dass sich in Primaten und Menschen bei infektiös bedingten Entzündungen das Enzym Glycin-N-Acyl-Transferase (GNAT) in beträchtlichen Konzentrationen nachweisen läßt, und zwar im Unterschied zu Gesunden, bei denen es nicht oder nur in Konzentrationen an der analytischen Nachweisgrenze gefunden wird, was die GNAT sowohl für die Entzündungsdiagnostik/Sepsisdiagnosik als auch als neues therapeutisches Target geeignet macht.

Die Verwendung in der Diagnose, die sich aufgrund des erstmals nachgewiesenen Auftretens von GNAT bei der experimentellen Simulation von Entzündungen bzw. Sepsis ergibt, wird in allgemeiner Form in Anspruch 1 beansprucht.

Die Ansprüche 2 bis 9 betreffen diagnostische Verwendungen bzw. Verfahren.

Wie nachfolgend im experimentellen Teil noch näher ausgeführt wird, beruht die Erfindung darauf, dass nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere ein nur bei den behandelten Tieren identifizierbares Peptid- bzw. Proteinprodukt gefunden werden konnte. Dieses spezifische Produkt wurde aus dem Elektrophoresegel isoliert und auf an sich bekannte Weise durch eine Sequenzanalyse des N-Terminus, die zur Identifizierung eines Teilpeptids von 8 Aminosäuren (SEQ ID NO:1) führte, und andererseits massenspektrometrisch untersucht. Es zeigte sich, dass das Teilpeptid vom N-Terminus in einer humanen cDNA-Datenbank in einem EST (GenBank Accession No. AB013093) vorkommt, der bei der Translation das Polypeptid mit der Aminosäuresequenz gemäss SEQ ID NO:2 ergibt. Aufgrund einer 76%igen Identität der genannen Aminosäuresequenz mit der bekannten Sequenz der bovinen GNAT (GenBank Accession No. AF045032) konnte das wie beschrieben gefundene Peptid als humane GNAT identifiziert werden.

In der WO 01/75067 A2, die 30368 Polynukleotidsequenzen und 30368 zugeordnete Peptid-Aminosäuresequenzen enthält, werden in allgemeiner Form mögliche, an sich bekannte, jedoch voneinander sehr verschiedenartige Verwendungen von Polynukleotiden und Peptiden beschrieben. Soweit die beschriebenen Sequenzen auch für GNAT relevante Sequenzen betreffen, ist dem Dokument keine brauchbare Lehre zu entnehmen, die eine Verwendung von GNAT als Biomarker für Sepsis und Entzündungen im Sinne der vorliegenden Erfindung betrifft.

Bei der massenspektrometischen Analyse des Proteinspots des Proteins aus der Pavianleber durch Tandem-Massenspektrometrie wurden zahlreiche kurze Peptidsequenzen gefunden, die sich überraschender Weise in identischer Form in keiner Sequenz aus dem humanen Genom wiederfanden. Nach der Identifizierung der humanen GNAT-Sequenz (SEQ ID NO:2) war es jedoch möglich, die mittels Massenspektrometrie identifizierten Sequenzen entsprechenden Teilsequenzen der humanen GNAT zuzuordnen. Es zeigte sich bei dem Vergleich der identifizierten Sequenzen aus dem Pavianprotein mit der Sequenz der humanen GNAT, dass bei den identifizierten Teilsequenzen, die alle im Bereich der Aminosäuren 140 bis 280 der humanen GNAT (SEQ ID NO.2) lagen, stets Deletionen oder ein Austausch von einer oder mehreren Aminosäuren festzustellen waren. Offensichtlich gehören die GNAT-Peptide zu den über die Familien- und Gattungsgrenzen hinweg stark variierenden, d.h. wenig konservierten Peptiden, worauf auch die erheblichen Unterschiede zwischen der gefundenen humanen GNAT und der bekannten bovinen GNAT hindeuten.

Aufgrund der Identität der durch Sequenzierung gefundenen Sequenz vom N-Terminus sowie der trotz der geschilderten Abweichungen hohen Homologie der massenspektrometrisch identifizierten Pavian-Teilsequenzen und humanen Teilsequenzen ist die Identifizierung des humanen Äquivalents zu dem isolierten Proteinspot als GNAT nach anerkannten Kriterien als eindeutig anzusehen.

Wenn in der vorliegenden Anmeldung das für diagnostische Zwecke bestimmte Peptid als "GNAT" bezeichnet wird, bedeutet das nicht, daß eine solche GNAT eine 100%ige Identität mit der Sequenz gemäss SEQ ID NO:2 aufweisen muss. Als GNAT wird in der vorliegenden Anmeldung vielmehr ein Peptid bezeichnet, das in der physiologisch vorkommenden Form an seinem N-Terminus eine Peptidsequenz gemäss SEQ ID NO:1 aufweist, und eine hohe Homologie, d.h. vorzugsweise von mehr als 60%, stärker bevorzugt 80%, zu der Sequenz der humanen GNAT gemäß SEQ ID NO:2 aufweist. Von dem Begriff GNAT werden auch Teilpeptide eines wie oben definierten Peptids erfasst, die insbesondere bei der Herstellung von Reagenzien, z.B. selektiven Antikörpern, für die GNAT-Bestimmung bzw. zur Herstellung von Assays zur GNAT-Bestimmung in biologischen Proben verwendet werden können. Als GNAT bzw. Teilsequenzen davon sind auch solche Sequenzen anzusehen, die nach Deletion von einer oder mehreren Aminosäuren oder kurzer Peptidsequenzen aus dem Peptid gemäss SEQ ID NO:2 erhalten werden. Ferner sind für diagnostische und/oder therapeutische Zwecke geeignete Teilsequenzen (Fragmente) insbesondere solche, die eine Folge von mindestens drei Aminosäuren, vorzugsweise mindestens 6 Aminosäuren, des Peptids SEQ ID NO:2 umfassen.

Für besondere diagnostische Zwecke können die erfindungsgemäßen GNAT-Peptide auch Säugetierpeptide sein, die eine wenigstens 60%ige Homologie zu dem Peptid mit SEQ ID NO:1 aufweisen sollten und z.B. für diagnostische Zwecke oder in der Veterinärmedizin verwendet werden.

Die nach anerkannten Kriterien als eindeutig anzusehende Identifizierung des nur nach Sepsis- bzw. Entzündungsauslösung in dem Pavian-Lebergewebe gefundenen Proteins als GNAT ist von hohem wissenschaftlichen, diagnostischen und therapeutischen Interesse. Als enzymatische Gewebe-Aktivität sind verschiedene Formen der GNAT schon lange bekannt. Eine Zuordnung der in der Datenbank vorhandenen cDNA-Sequenz zur humanen GNAT ist nach diesseitiger Kenntnis noch nicht erfolgt. Die GNAT-Aktivität ist Gegenstand von Untersuchungen mit primär wissenschaftlicher Zielsetzung. Es können hier beispielsweise genannt werden Veröffentlichungen von Margaret O. James et al., Biochem.J. (1978) 172, 285-291; M. Kelley et al., J.Biochem.Toxicology, Vol.8, No.2, pp. 63-69 (1993); Yogesh R. Marwal et al., Biochem.Biophys.Res.Comm. Vol.205, No.2, 1994, pp. 1373-1379; D.J.Merkler et al., Archives of Biochemistry and Biophysics, Vol.330, No.2, pp. 430-434, 1996; Yogesh R. Marwal et al., J Pediatr 1997, 130, 1003-1007; Francois H. van der Westhuizen et al., J.Biochem. Toxicol 14:102-109, 2000. In den genannten Arbeiten wird GNAT in erster Linie als Enzym diskutiert, das durch die Peptidbindungs-Konjugation von endogenen und exogenen Carbonsäuren eine wichtige Rolle bei der Entgiftung des Organismus von Säugetieren spielt. Für die medizinische Diagnostik und Therapie spielte GNAT bisher keine Rolle.

Mit der vorliegenden Erfindung wird einerseits, gemäß Anspruch 1, Schutz für die darin genannten Verwendungen eines Peptids begehrt, das als GNAT bezeichnet wird und gemäss der o.g. Definition primär durch die Anwesenheit der Teilsequenz aus 8 Aminosäuren gemäß SEQ ID NO:1 definiert ist und dadurch von allen anderen bekannten humanen Peptiden und Proteinen unterscheidbar ist, ohne dass bezüglich der Länge und Natur weiterer Aminosäuresequenzen Einschränkungen bestehen sollen.

Aufgrund der nunmehr bekannten Sequenz und der physiologischen Rolle der humanen GNAT in Verbindung mit den Befunden zu ihrer verstärkten Bildung bei Entzündungen und Sepsis können die humane GNAT bzw. Teile davon für diagnostische und/oder therapeutische Zwecke nach Verfahren, die inzwischen zum Stand der Technik gehören, synthetisch oder gentechnologisch als Rekombinationsprodukte hergestellt werden.

Ferner können die GNAT-Peptide nach bekannten Verfahren des modernen Standes der Technik auch zur Erzeugung spezifischer polyklonaler und insbesondere monoklonaler Antikörper verwendet werden, die als Hilfsmittel für die diagnostische Bestimmung der erfindungsgemäßen Peptide und/oder auch als potentielle therapeutische Mittel geeignet sind. Die Erzeugung geeigneter monoklonaler Antikörper gegen bekannte Peptid-Teilsequenzen gehört heute zum allgemeinen Stand der Technik.

Bei der Bestimmung der GNAT oder von ausgewählten Teilpeptiden davon kann dabei grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; wobei ausdrücklich ergänzend auch auf die dort beschriebenen Immunisierungstechniken verwiesen wird, die eine Möglichkeit für die Gewinnung von monoklonalen Antikörpern auch gegen Teilsequenzen der GNAT darstellen. Variationen der beschriebenen Techniken und/oder weitere Immunisierungstechniken kann der Fachmann einschlägigen Standardwerken und Veröffentlichungen entnehmen und sinngemäß anwenden.

Ferner ist auch ausdrücklich die Erzeugung von GNAT-Antikörpern unter Anwendung von Techniken der direkten genetischen Immunisierung mit DNA zu erwähnen. Es liegt ferner im Rahmen der vorliegenden Erfindung, zur Immunisierung z.B. eine cDNA der gewünschten GNAT oder ihrer Teilpeptide zu verwenden, da es sich in der Vergangenheit gezeigt hat, dass durch derartige Immunisierungstechniken das Spektrum der gewinnbaren Antikörper erweitert werden kann. GNAT gemäß SEQ ID NO:2 oder Teilpeptide davon, z.B. solche, die die Teilsequenz SEQ ID NO:1 und/oder andere Teilsequenzen enthalten, können aufgrund der vorliegenden Ergebnisse als spezifische Markerpeptide (Biomarker) zum diagnostischen Nachweis und zur Verlaufskontrolle von Entzündungen und Infektionen (insbesondere auch von systemischen Infektionen vom Sepsistyp) dienen. Wie die Bestimmung von Procalcitonin kann dabei die Bestimmung der GNAT durch die Anwendung eines Verfahrens zur differentialdiagnostischen Früherkennung und zur Erkennung sowie zur Beurteilung des Schweregrads und zur therapiebegleitenden Verlaufsbeurteilung von Sepsis und Infektionen erfolgen, wobei man bei einem derartigen Verfahren in einer Probe einer biologischen Flüssigkeit oder eines Gewebes eines Patienten den Gehalt an GNAT oder eines Teilpeptids davon bestimmt und aus der festgestellten Anwesenheit und/oder Menge des bestimmten Peptids auf das Vorliegen einer Entzündung, einer schweren Infektion oder einer Sepsis schließt und das erhaltene Ergebnis mit dem Schweregrad der Sepsis korreliert und die Behandlungsmöglichkeiten und/oder die Behandlungsaussichten abschätzt.

An Stelle der Bestimmung der GNAT oder ihrer Bruchstücke oder ggf. posttranslational modifizierten Formen davon ist für diagnostische Zwecke auch die Bestimmung der zugehörigen mRNA möglich. Die GNAT-Bestimmung kann für diagnostische Zwecke auch indirekt als Bestimmung ihrer Enzymaktivität in einem entzündeten Organ oder Gewebe oder einer biologischen Flüssigkeit erfolgen.

Es ist ferner möglich, die Bestimmung von GNAT als Prognosemarker und Marker für die Überwachung der Krankheitsverlaufs von Entzündungen, insbesondere systemischen Entzündungen, und Sepsis im Rahmen einer Kombinationsmessung mit anderen Markern durchzuführen.

Neben einer Kombination mit einer Procalcitoninmessung kommt insbesondere eine Kombination der Messung von GNAT mit der Bestimmung anderer Marker für Sepsis und systemische Entzündungen in Betracht, und zwar insbesondere mit CA 19-9, CA 125, S100B oder an der Regulierung von Entzündungen beteiligten S100A-Proteinen, oder mit der Bestimmung der in den älteren, nachfolgend genannten unveröffentlichten deutschen Patentanmeldungen der Anmelderin beschriebenen neuartigen Sepsismarker Inflammin (DE 101 19 804.3) und CHP (DE 101 31 922.3), und/oder mit der Bestimmung von löslichen Cytokeratinfragmenten, insbesondere der neu aufgefundenen löslichen Cytokeratin-1-Fragmente (sCY1F;; DE 101 30 985.6) sowie der bekannten Tumormarker CYFRA-21 oder TPS und/oder eines oder mehrerer der o.g. Prohormone. Auch eine gleichzeitige Bestimmung des bekannten Entzündungsparameters C-reaktives Protein (CRP) kann vorgesehen sein. Aufgrund der in dieser und den parallelen Anmeldungen beschriebenen neuen Ergebnisse ist für die Sepsis-Feindiagnose außerdem generell eine Kombination mit Messungen von bekannten oder noch aufzufindenden Biomolekülen in Betracht zu ziehen, die als gewebe- bzw. organspezifische Entzündungsmarker geeignet sind.

GNAT oder ihre Bruchstücke oder Fusionsprodukte oder die dafür kodierende DNA können auch in der präventiven Medizin oder Therapie verwendet werden. So können z.B. geeignete GNAT-Bruchstücke zur in-vivo-Erzeugung von GNAT-bindenden Antikörpern durch aktive Immunisierung nach an sich bekannten Techniken verwendet werden. Als GNAT sollen dabei auch solche Moleküle anzusehen sein, die die vollständige GNAT oder geeignete Teilsequenzen davon in posttranslational modifizierter Form, z.B. in glykosylierter oder phosphorylierter Form, oder auch in einer durch pharmazeutische Hilfsstoffe, z.B. Polyethylenglykolreste, substituierten Form enthalten. GNAT oder geeignete Teilsequenzen davon können auch in dem Sinne als Target für therapeutische Interventionen dienen, dass mittels geeigneter spezifischer Binder für GNAT oder Teilpeptide davon GNAT introkorporal inaktiviert wird oder ggf. auch extrakorporal im Sinne einer "Blutwäsche" bzw. Plasmapherese unter Verwendung geeigneter Immunadsorbentien bzw. mit spezifischen Bindern für GNAT beschichteten perfundierbaren Festphasen aus dem Kreislauf von Patienten eliminiert wird.

Zur in-vivo-Inaktivierung von GNAT kommen insbesondere spezifische Antikörper, insbesondere humanisierte monoklonale Antikörper, in Frage. Die therapeutische Beeinflussung der Entzündungskaskade kann aber auch unter Einsatz der GNAT selbst oder von GNAT-Agonisten oder -Antagonisten erfolgen. Derartige therapeutische Interventionen werden insbesondere dann möglich, wenn weitere Erkenntnisse zur physiologischen Funktion der GNAT im Rahmen eines Entzündungsgeschehens abgesichert sind. So erscheint es derzeit nicht ausgeschlossen, dass GNAT im Entzündungsgeschehen eine wichtige Rolle spielt, möglicherweise durch direkte oder indirekte Beeinflussung des Krankheitsgeschehens auf dem Wege der Acylierung von Peptiden, Proteinen, Lipiden, Zuckermolekülen und anderen Substanzen.

Nachfolgend wird die Auffindung und Identifizierung der GNAT in näheren Einzelheiten geschildert, wobei auf das beigefügte Sequenzprotokoll bezug genommen wird. Die Figuren zeigen:
- Fig. 1: Ansichten von 2D-Elektrophoresegelen, die einen Vergleich der Spotmuster von cytoplasmatischen Leberzellprotein eines gesunden Pavians (A) mit den Leberzellproteinen eines Pavians 5h nach einer durch LPS-Verabreichung induzierten Sepsis (B) ermöglichen. Der Pfeil zeigt die Position des erfindungsgemäßen sepsisspezifischen Produkts GNAT an, das in Darstellung (B) durch einen Kreis hervorgehoben ist;
- Fig. 2: das Massenspektrum des durch 2D-Gelelektrophorese identifizierten Proteinspots aus der Pavianleber (Pavian-GNAT) nach dessen Trypsinverdauung.

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

Weil Versuche mit den wie oben gewonnenen Proben ergaben, dass die größte Procalcitoninmenge in Lebergewebe behandelter Tiere gefunden wird, wurde für die Suche nach neuen sepsisspezifischen Biomarkern mit Proteinextrakten aus der Pavianleber gearbeitet.

### 2. Proteomanalyse unter Verwendung cytoplasmatischer Leberzellproteine von Pavianen.

Cytoplasmatische Leberzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Leberextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

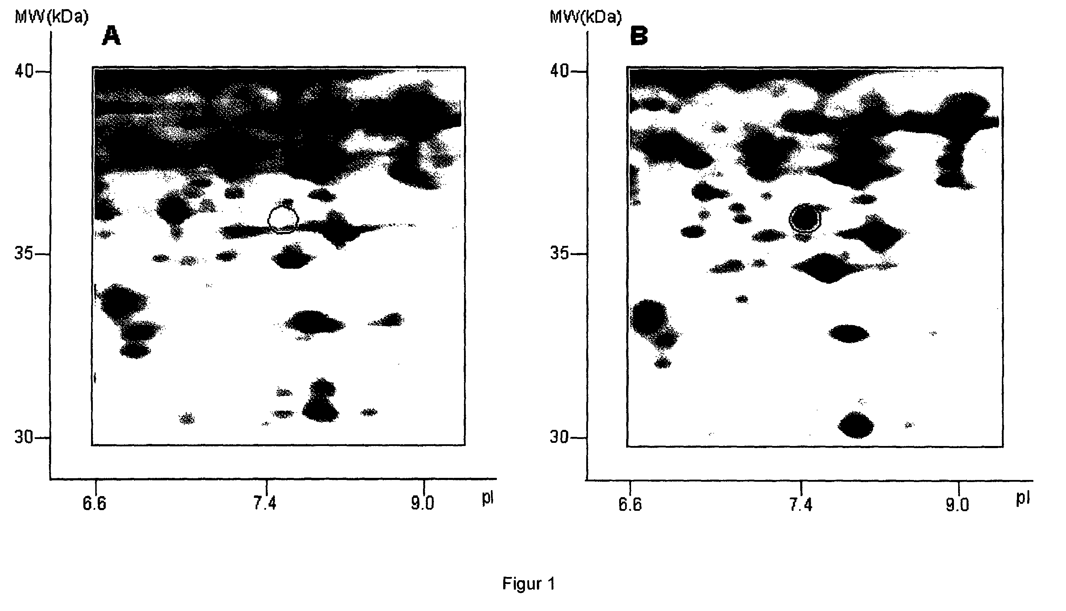

Zur Auswertung wurden die Proteinspotmuster der Proben unbehandelter Tieren mit den Proteinspotmustern verglichen, die aus Lebergewebeproben behandelter Tiere resultierten. Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei der zusätzliche Proteinspot in (B) GNAT entspricht, deren Position durch einen Pfeil und einen Kreis hervorgehoben ist.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Der für die weitere Analyse vorselektierte Proteinspot wurde aus dem Gel ausgeschnitten.

Zur N-terminalen Sequenzierung wird der separierte Proteinspot mittels Elektroblotting auf PVDF-Membranen nach dem Verfahren, das von P.Matsudaira in: J.Biol.Chem. Vol. 262, No.21, pp. 10035-10038, 1987 beschrieben wurde, transferiert. Das auf PVDF-Membranen immobilisierte Protein wurde dann mittel automatischen Edman-Abbaus unter Verwendung eines ProCise® Sequenzers, ausgestattet mit einem 140 C Mikrogradientensystem und einem 785 programmierbaren Detektor (PE-Applied Biosystems) nach den Vorschriften des Herstellers endsequenziert. Dabei wurde für den in Figur 1B gekennzeichneten Proteinspot ein Peptid aus 8 Aminosäuren identifiziert, das die in SEQ ID NO:1 gezeigt Sequenz aufweist.

Ferner wurde der Proteinspot unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden. Dabei wurden die trypsinverdauten Proben nach einer ESI (ElectroSprayIonisierung) einer Tandem-Massenspektrometrie unterzogen. Es wurde ein Q-TOF-Massenspektrometer mit einer sog. nanoflow-Z-Spray-Ionenquelle der Firma Micromass, UK, verwendet. Dabei wurde entsprechend der Arbeitsanleitung des Geräteherstellers gearbeitet.

### 3. Identifizierung von GNAT

Wie in den Figuren 1(A) und 1(B) gezeigt ist, findet sich in Leberzellextrakten von Pavianen, denen eine LPS-Injektion verabreicht worden war, u.a. ein neues Protein, für das aufgrund der Gelektrophoresedaten im Vergleich mit Markersubstanzen mit bekanntem Molekulargewicht ein Molekulargewicht von ca. 37000 ± 700 Dalton abgeschätzt wurde, während aus der relativen Position des Proteins aus der ersten Dimension ein isoelektrischer Punkt von ca. 7,0 bis 8,0 abgeschätzt wurde.

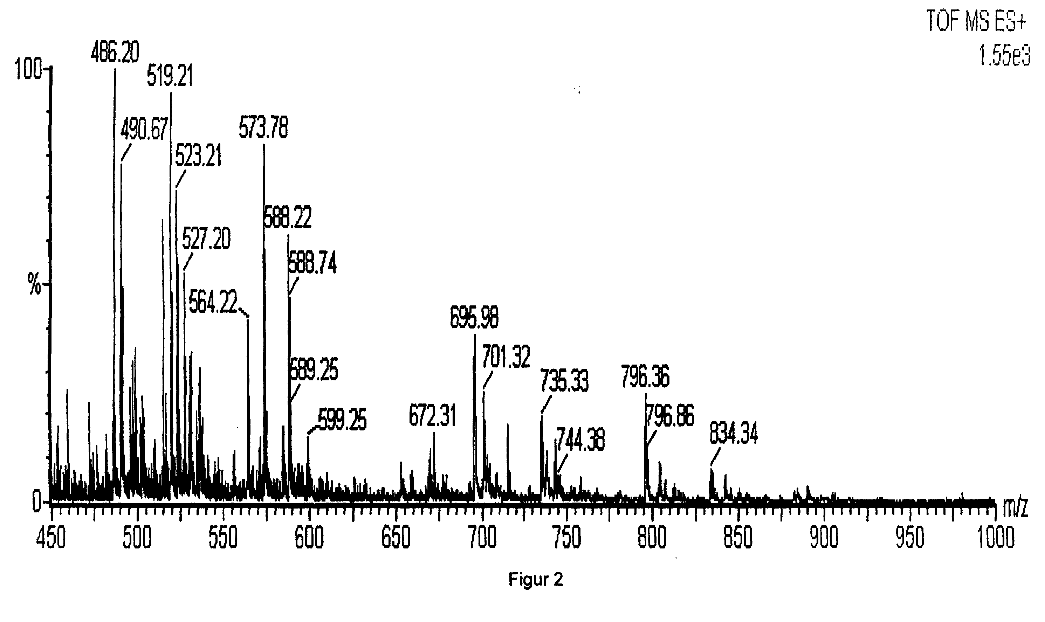

Dieses Protein wurde wie oben beschrieben einer Sequenzanalyse seines N-Terminus unterzogen. Ferner wurde es massenspektrometrisch analysiert, wobei Fig. 2 das Massenspektrum des gesamten trypsinverdauten Proteins zeigt.

Fragmente des "Mutterspektrums" gemäß Fig. 2 wurden durch Tandem-Massenspektroskopie identifiziert. Die für diese Fragmente erhaltenen Massenspektrum konnte auf sich bekannte Weise rechnerisch als Peptid-Teilsequenzen (nicht gezeigt) identifiziert werden, die im humanen Genom keine direkte Entsprechung fanden, aber aufgrund einer vorhandenen partiellen Sequenzidentität bestimmten Abschnitten der humanen GNAT gemäss SEQ ID NO:2 zugeordnet werden konnten.

Die durch Sequenzierung des N-Terminus identifizierte Teilsequenz gemäß SEQ ID:NO:1 wurde dann mit Proteinsequenzen verglichen, die in Sequenzdatenbanken verfügbar waren. Mittels eines Programms FASTA3 (http://www2.ebi.ac.uk/fasta3/) wurde die Sequenz SEQ ID NO:1 in der Datenbak "swall" im Eintrag 014833 gefunden. Der entsprechnde GenBank-Eintrag lautet AF023466. Mittels eines Programms BLAST wurde in der Datenbank est_human dann die Sequenz mit der GenBank Accession No. AB013093 identifiziert, die bei ihrer Translation die Aminosäuresequenz SEQ ID NO:2 liefert. Aufgrund ihrer 76%igen Identität mit der bekannten bovinen GNAT-Sequenz (GenBank Accession No. AF045032) wurde die gefundene Aminosäuresequenz als die der humanen GNAT identifiziert.

Die damit explizit bekannte vollständige Sequenz der humanen GNAT gestattet es, für humanmedizinische Zwecke (Diagnostik oder Therapie) humane GNAT oder beliebige Teilsequenzen (Fragmente) davon gezielt herzustellen, und zwar unter Anwendung bekannter synthetischer oder gentechnologischer Verfahren zur Herstellung von Peptiden. Für veterinärmedizinische Zwecke gilt entsprechendes, wobei in diesen Fällen auf entsprechende bekannte, z.B. bovine, GNAT-Sequenzen zurückgegriffen werden kann, oder tierspezifische GNAT-Sequenzen in entsprechenden tierspezifischen Datenbanken aufgrund der Analogien mit den bekannten bovinen und humanen Sequenzen leicht aufgefunden werden können. Derartige Peptide können dann, beispielsweise in Analogie zu der in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; beschriebenen Arbeitsweise zur Schaffung von geeigneten Antikörpern, insbesondere monoklonalen Antikörpern dienen, die ihrerseits die Schaffung von Assays für die immundiagnostische Bestimmung der GNAT oder ausgewählter Teilpeptide davon ermöglichen.

Auf die skizzierte bekannte Weise erhältliche monoklonale Antikörper können auch, insbesondere nach einer an sich bekannten Humanisierung, der Entwicklung von neuen Therapeutika dienen (vgl. die in K.Garber, "Protein C may be sepsis solution", Nature Biotechnology, Vol.18, 2000, 917-918; zusammengefassten therapeutischen Ansätze). Ferner ist eine in-vivo-Neutralisierung der GNAT auch durch Blockade der Expression des GNAT-Gens möglich. Zu den sich aufgrund der Erkenntnisse in der vorliegenden Anmeldung ergebenden therapeutischen Interventionen gehört auch die Verabreichung von Wirkstoffen, die die Enzymaktivität der GNAT hemmen.

## Patentansprüche

1. Verwendung der Glycin-N-Acyl-Transferase (GNAT) aus Körperflüssigkeiten oder Körpergeweben in der Human- und Tiermedizin als Markerpeptid zum diagnostischen Nachweis *in vitro*, für die Verlaufsprognose und für die Verlaufskontrolle von Entzündungen und Infektionen.

2. Verwendung von GNAT nach Anspruch 1 im Rahmen der differentialdiagnostischen Früherkennung und Erkennung für die Verlaufsprognose, die Beurteilung des Schweregrads und für die therapiebegleitende Verlaufsbeurteilung von Sepsis und schweren Infektionen, insbesondere sepsisähnlichen systemischen Infektionen, durch *in vitro* Bestimmung des Auftretens und/oder der Menge von GNAT in einer biologischen Flüssigkeit oder einer Gewebeprobe eines Patienten.

3. *In vitro*-Verfahren zur differentialdiagnostischen Früherkennung und Erkennung, für die Verlaufsprognose und die Beurteilung des Schweregrads und zur therapiebegleitenden Verlaufsbeurteilung von Sepsis und schweren Infektionen, insbesondere sepsisähnlichen systemischen Infektionen, **dadurch gekennzeichnet, dass** man die Anwesenheit und/oder Menge von GNAT in einer biologischen Flüssigkeit oder einer Gewebeprobe eines Patienten bestimmt und aus der bestimmten Anwesenheit und/oder Menge von GNAT Schlüsse hinsichtlich des Vorliegens, des zu erwartenden Verlaufs, des Schweregrads oder den Erfolg eines Therapie der Sepsis oder von Infektion zieht.

4. Verfahren nach Anspruch 3, **dadurch gekennzeichnet, dass** es ein immundiagnostisches Bestimmungsverfahren ist.

5. Verfahren nach Anspruch 3, **dadurch gekennzeichnet, dass** Bestimmung der GNAT indirekt als Bestimmung der zugehörigen GNAT-mRNA oder der GNAT-Enzymaktivität erfolgt.

6. Verfahren nach einem der Ansprüche 3 bis 5, **dadurch gekennzeichnet, dass** es im Rahmen einer Multiparameter-Bestimmung durchgeführt wird, bei der gleichzeitig mindestens, ein weiterer Sepsisparameter bestimmt wird und ein Messergebnis in Form eines Satzes von mindestens zwei Messgrößen gewonnen wird, der zur Sepsis-Feindiagnostik ausgewertet wird.

7. Verfahren nach Anspruch 6, **dadurch gekennzeichnet, dass** im Rahmen der Multiparameter-Bestimmung neben GNAT wenigstens ein weiterer Parameter bestimmt wird, der ausgewählt ist aus der Gruppe, die besteht aus Procalcitonin, CA 19-9, CA 125, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen und dem C-reaktiven Protein (CRP).

8. Verfahren nach Anspruch 6 oder 7, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

9. Verfahren nach Anspruch 8, **dadurch gekennzeichnet, dass** die Auswertung des mit der Messvorrichtung gewonnenen komplexen Messergebnisses mit Hilfe eines Computerprogramms erfolgt.

## Claims

1. Use of glycine N-acyltransferase (GNAT) from body fluids or body tissues in human and veterinary medicine as a marker peptide for *in vitro* diagnosis, for prognosis and for monitoring the course of inflammations and infections.

2. Use of GNAT according to Claim 1 in early differential diagnosis and detection, for prognosis, for assessing the severity and for the therapy-accompanying assessment of the course of sepsis and severe infections, in particular sepsis-like systemic infections, by *in vitro* determination of the occurrence and/or the amount of GNAT in a biological fluid or a tissue sample of a patient.

3. *In vitro* method for early differential diagnosis and detection, for prognosis and assessment of the severity and for the therapy-accompanying assessment of the course of sepsis and severe infections, in particular sepsis-like systemic infections, **characterized in that** the presence and/or the amount of GNAT in a biological fluid or a tissue sample of a patient is determined and conclusions are drawn with regard to the presence, the expected course, the severity or the success of a treatment of the sepsis or of infection from the determined presence and/or amount of GNAT.

4. Method according to Claim 3, **characterized in that** it is an immunodiagnostic assay method.

5. Method according to Claim 3, **characterized in that** the determination of GNAT is effected indirectly as a determination of the associated GNAT-mRNA or of the GNAT enzymatic activity.

6. Method according to any of Claims 3 to 5, **characterized in that** it is carried out in the course of a multiparameter determination in which simultaneously at least one further sepsis parameter is determined and a measured result is obtained in the form of a set of at least two measured variables which is evaluated for fine diagnosis of sepsis.

7. Method according to Claim 6, **characterized in that**, in the course of multiparameter determination, at least one further parameter which is selected from the group consisting of procalcitonin, CA 19-9, CA 125, S100B, S100A proteins, soluble cytokeratin fragments, in particular CYFRA 21, TPS and/or soluble cytokeratin-1 fragments (sCY1F), the peptides inflammin and CHP, peptide prohormones and the C-reactive protein (CRP) is determined in addition to GNAT.

8. Method according to Claim 6 or 7, **characterized in that** the multiparameter determination is carried out as a simultaneous determination by means of a chip technology measuring apparatus or an immunochromatographic measuring apparatus.

9. Method according to Claim 8, **characterized in that** the evaluation of the complex measured result obtained using the measuring apparatus is effected with the aid of a computer program.

## Revendications

1. Utilisation de la glycine-N-Acyl transférase (GNAT) en provenance de volume humoral ou de tissus, dans la médecine humaine ou vétérinaire, en tant que peptide marqueur pour le diagnostique in vitro, pour le pronostic concernant l'évolution et pour le contrôle de l'évolution d'inflammations et d'infections.

2. Utilisation de GNAT selon la revendication 1 dans le cadre de la détection précoce et de la détection pour le pronostic concernant l'évolution, l'évaluation du degré de gravité et pour l'évaluation de l'évolution, accompagnant la thérapie, de septicémie et d'infections graves, notamment d'infections systémiques, analogues à la septicémie, par détermination in vitro de l'apparition et / ou de la quantité de GNAT dans un liquide biologique ou un prélèvement de tissu d'un patient.

3. Procédé in vitro pour la détection précoce et la détection par diagnostic différentiel, pour le pronostic concernant l'évolution et l'évaluation du degré de gravité, et pour l'évaluation de l'évolution accompagnant la thérapie de septicémie et d'infections graves, notamment d'infections systémiques, analogues à la septicémie,
**caractérisé en ce que** l'on détermine la présence et / ou la quantité de GNAT dans un liquide biologique ou dans un prélèvement de tissu d'un patient, et que l'on tire, sur la base de la présence et / ou de la quantité d'un fragment déterminé, des conclusions quant à la présence, à l'évolution à attendre, au degré de gravité ou au succès d'une thérapie de la septicémie ou de l'infection.

4. Procédé selon la revendication 3, **caractérisé en ce que** celui-ci est un procédé de définition diagnostique immunologique.

5. Procédé selon la revendication 3, **caractérisé en ce que** la définition de la GNAT est effectuée indirectement en tant que définition de l'ARNm de la GNAT associée ou de l'activité enzymatique GNAT.

6. Procédé selon l'une des revendications 3 à 5, **caractérisé en ce que** celui-ci est réalisé dans le cadre d'une détermination de paramètres multiples, lors de laquelle au moins un autre paramètre de septicémie est simultanément déterminé, et un résultat de mesure est obtenu sous la forme d'un jeu d'au moins deux grandeurs de mesure, lequel est exploité pour le diagnostic précis de la septicémie.

7. Procédé selon la revendication 6, **caractérisé en ce que**, dans le cadre d'une détermination multi-paramètres, outre la GNAT, au moins un autre paramètre est déterminé, lequel est sélectionné dans le groupe comprenant procalcitonine, CA 19-9, CA 125, s100B, protéines S100A, fragments de cytokératine, solubles, notamment CYFRA 21, TPS et / ou fragments de cytokératine 1 sCY1F), les peptides inflammine et CHP, prohormones peptidiques et la protéine réactive C (CRP).

8. Procédé selon la revendication 6 ou 7, **caractérisé en ce que** la détermination multi-paramètres est effectuée en tant que détermination simultanée au moyen d'un dispositif de mesure de technologie à puce ou un dispositif immuno-chromatographique.

9. Procédé selon la revendication 8, **caractérisé en ce que** l'évaluation du résultat de mesures obtenu au moyen du dispositif de mesure est effectuée à l'aide d'un programme informatique.
